(19) Europäisches Patentamt
European Patent Office
Office européen des brevets

(11) **EP 2 623 033 B1**

(12) **EUROPEAN PATENT SPECIFICATION**

(45) Date of publication and mention
of the grant of the patent:
**11.01.2017 Bulletin 2017/02**

(51) Int Cl.:
*A61B 8/00* *(2006.01)*  *A61B 8/08* *(2006.01)*
*A61B 5/107* *(2006.01)*  *A61B 8/14* *(2006.01)*
*A61B 5/00* *(2006.01)*  *G06F 19/00* *(2011.01)*

(21) Application number: **11828374.6**

(22) Date of filing: **26.09.2011**

(86) International application number:
**PCT/JP2011/005365**

(87) International publication number:
**WO 2012/042808 (05.04.2012 Gazette 2012/14)**

(54) **ULTRASOUND DIAGNOSTIC APPARATUS**

ULTRASCHALLDIAGNOSEGERÄT

ÉQUIPEMENT DE DIAGNOSTIC PAR ULTRASONS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.09.2010 JP 2010222568**

(43) Date of publication of application:
**07.08.2013 Bulletin 2013/32**

(73) Proprietor: **Konica Minolta, Inc.
Tokyo 100-7015 (JP)**

(72) Inventor: **TOJI, Bunpei
Osaka-shi, Osaka 540-6207 (JP)**

(74) Representative: **Wenning, Ekkehard et al
Henkel, Breuer & Partner
Patentanwälte
Maximiliansplatz 21
80333 München (DE)**

(56) References cited:
**JP-A- 2001 198 122   JP-A- 2008 136 860
US-A1- 2007 081 705   US-B1- 6 375 616
US-B1- 6 575 907**

**Description**

[Technical Field]

The present invention relates to ultrasound diagnostic apparatuses, and particularly relates to an ultrasound diagnostic apparatus used for examination on the growth of a fetus.

[Background Art]

Ultrasound-based diagnostic imaging, by its nature of utilizing sound waves, affects less the human body. Therefore, the ultrasound-based diagnostic imaging is often used for prenatal checkups, and the condition in which a fetus grows is examined with reference to the ultrasound images of the fetus during a checkup.

For the examination on the condition of a growing fetus, it is a well-known method to calculate an estimated weight of the fetus based on the ultrasound images. More specifically, the estimated fetal weight is calculated by measuring the lengths of specific regions (head, abdomen, and thigh) of the fetus in the mother's uterus and substituting the measured values into a formula used for the estimation of the fetal weight.

In the general operation performed in the ultrasound-based diagnostic imaging, the examiner firstly operates a probe in such a manner that the specific regions of a fetus are delineated. Then, the examiner adjusts the probe so that the cross-sectional images which are appropriate for the use in the measurement can be obtained, and allows the measurement images of the specific regions to be displayed. The examiner then measures, on the respective measurement images, a biparietal diameter (BPD) for the head, an abdominal circumference (AC) for the abdomen, and a femoral length (FL) for the thigh, of the fetus. The estimated fetal weight can be obtained by inputting the values which have resulted from the respective measurements into the estimated fetal weight calculation formula as shown in Formula 1 below.

$$\text{Estimated weight (g)} = 1.07\text{BPD}^3 + 3.00 \times 10^{-1}\text{AC}^2 \times \text{FL} \quad (\text{Formula 1})$$

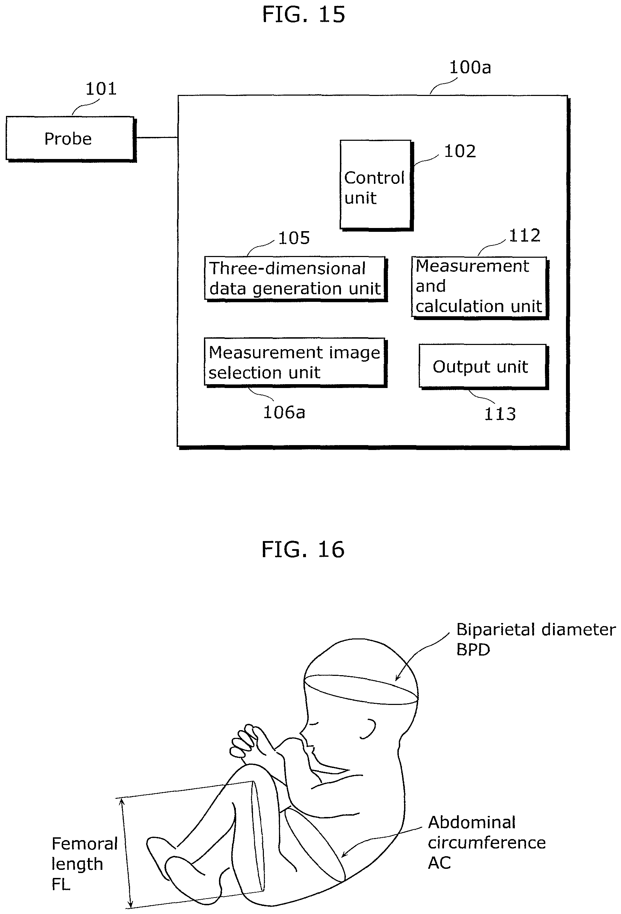

Here, BPD (biparietal diameter/cm), AC (abdominal circumference/cm), and FL (femoral length/cm) are the lengths of the regions respectively shown in FIG. 16. FIG. 16 is a diagram illustrating the specific regions of a fetus which are used for the estimated fetal weight calculation formula.

According to such conventional method, an estimated fetal weight can be obtained by measuring the lengths of the BPD, the AC, and the FL after the respective appropriate measurement images (hereafter referred to as "measurement reference images") have been displayed. Then, by comparing the estimated fetal weight thus obtained and the statistical data of estimated fetal weight, it is possible to examine the condition of a growing fetus.

With the conventional method, however, in the case where the measurement reference images are inappropriate, that is, the case in which the respective measurement reference images are not displayed in an appropriate manner so as to measure the lengths of the BPD, the AC, and the FL, it is impossible to accurately measure these lengths. For example, in the case of displaying a thighbone in the thigh, the thighbone may be displayed with the length shorter than its actual length on the measurement reference image if the angle between the probe and the thighbone is not appropriate. The same applies to the head and the abdomen, and the lengths of the biparietal diameter and the abdominal circumference may be displayed with the lengths longer than their actual lengths depending on the angle that is respectively made with the probe.

Therefore, in order to properly obtain an estimated fetal weight, the examiner has to operate the probe carefully so as to obtain appropriate measurement reference images and thus determine appropriate measurement reference images. In other words, whether or not an estimated fetal weight can be properly obtained (whether the measurement reference images determined by the examiner enable accurate measurements of the BPD, the AC, and the FL) depends on the skills and knowledge of the examiner. This is attributed to the fact that the location and the position of a fetus always change during the examination.

In response to this problem, there is disclosed a technique of obtaining voxel data that compose a three-dimensional region, through the transmission and reception of ultrasound waves, and setting a cut plane for the voxel data so as to obtain cross-sectional images at arbitrary angles (see reference to PTL1). With the use of the method suggested in PTL1 for the obtainment of the measurement reference images as described above, the examiner is capable of setting appropriate cut planes after having obtained the voxel data of a fetus during the operation of the probe. In other words, it is possible to set appropriate measurement reference images regardless of the skills of the examiner.

[Citation List]

[Patent Literature]

[PTL 1] Japanese Unexamined Patent Application Publication No. H9-308630

US 6 575 907 B1 discloses an apparatus for measuring the weight of a fetus in utero including an ultrasonic imager providing at least one ultrasonic image, a volume determiner operative to employ the at least one ultrasonic image to provide volume information relating to at least part of the volume of the fetus in utero, and a weight determiner operative to employ said volume information relating to at least part of the volume of the fetus and density information relating to the at least part of the volume of the fetus for providing an output indication representing the weight of the fetus in utero.

[Summary of Invention]

[Technical Problem]

However, with the conventional configuration using the technique disclosed in the aforementioned PTL 1, although the influence caused by the dependence on the examiner's skills is reduced, the examiner needs to set cut planes, and thus, whether or not appropriate measurement reference images can be obtained still depends on the judgments of the examiner. That is to say, the problem, which is caused by the fact that the examiner has to judge whether the respective measurement reference images are appropriate for the measurements and has to give instructions based on the judgments, still remains to be solved.

[Solution to Problem]

The present invention solves the aforementioned conventional problem and has an object to provide an ultrasound diagnostic apparatus capable of reducing the dependence on the examiner and calculating an estimated fetal weight with high accuracy and easy operation.

In order to solve the conventional problem, there is provided an ultrasound diagnostic apparatus, as set out in independent claim 1, and an image processing method, as set out in independent claim 11.

With this configuration, it is possible to realize the ultrasound diagnostic apparatus capable of reducing the dependence on the examiner and calculating an estimated fetal weight with high accuracy and easy operation. Further advantageous developments are defined in the remaining dependent claims.

With this configuration, it is possible to select, with high accuracy, a cross-section appropriate for measurement by narrowing down the number of appropriate cut planes based on the three-dimensional features of a hyperechoic region so as to obtain an appropriate cut plane.

It should be noted that the present invention may be implemented, not only as an ultrasound diagnostic apparatus such as that described herein, but also as a method, having as steps, the processing units configuring the ultrasound diagnostic apparatus, and also as a program which causes a computer to execute such characteristic steps, and even as information, data or a signal which indicates the program. In addition, such a program, information, data, and signal can be distributed via a recording medium such as a CD-ROM and via a transmitting medium such as the Internet.

[Advantageous Effects of Invention]

According to the present invention, it is possible to realize an ultrasound diagnostic apparatus capable of reducing the dependency on the examiner and calculating an estimated fetal weight with high accuracy and easy operation.

[Brief Description of Drawings]

[FIG. 1] FIG. 1 is a block diagram showing an outline of an ultrasound diagnostic apparatus according to Embodiment 1 of the present invention.

[FIG. 2] FIG. 2 is a pattern diagram of previously-prepared template data which represents three-dimensional features of an abdomen of a fetus, according to Embodiment 1 of the present invention.

[FIG. 3] FIG. 3 is a pattern diagram of previously-prepared template data which represents three-dimensional features of a head of a fetus, according to Embodiment 1 of the present invention.

[FIG. 4] FIG. 4 is a pattern diagram of previously-prepared template data which represents three-dimensional features of a thigh of a fetus, according to Embodiment 1 of the present invention.

[FIG. 5] FIG. 5 is a pattern diagram for describing features of a measurement cross-section to be used for a measurement of a biparietal diameter (BPD) in the head of a fetus.

[FIG. 6] FIG. 6 is a pattern diagram for describing features of a measurement cross-section to be used for a measurement of an abdominal circumference (AC) in the abdomen of a fetus.

[FIG. 7A] FIG. 7A is a pattern diagram for describing features of a measurement cross-section to be used for a measurement of a femoral length (FL) in the thigh of a fetus.

[FIG. 7B] FIG. 7B is a diagram schematically showing a measurement cross-section with which the FL of a fetus is measured incorrectly.

[FIG. 8] FIG. 8 is a flowchart for describing a measurement reference image selection process performed by the ultrasound diagnostic apparatus according to Embodiment 1 of the present invention.

[FIG. 9] FIG. 9 is a flowchart for describing the processing that is up to the process of calculating an estimated weight of a subject, according to Embodiment 1 of the present invention.

[FIG.10] FIG. 10 is a flowchart showing a measurement reference image selection process performed for the head of a fetus by the ultrasound diagnostic apparatus according to Embodiment 1 of the present invention.

[FIG.11] FIG. 11 is a flowchart showing a measurement reference image selection process performed for the abdomen of a fetus by the ultrasound diagnostic apparatus according to Embodiment 1 of the present invention.

[FIG.12] FIG. 12 is a flowchart showing a measurement reference image selection process performed for the thigh of a fetus by the ultrasound diagnostic apparatus according to Embodiment 1 of the present invention.

[FIG.13] FIG. 13 is a block diagram showing an outline of an ultrasound diagnostic apparatus according to Embodiment 2 of the present invention.

[FIG.14] FIG. 14 is a flowchart for describing a measurement reference image selection process performed by the ultrasound diagnostic apparatus according to Embodiment 2 of the present invention.

[FIG.15] FIG. 15 is a diagram showing a minimal configuration of the ultrasound diagnostic apparatus according to the present invention.

[FIG.16] FIG. 16 is a diagram showing specific regions of a fetus which are used for an estimated fetal weight calculation formula.

[Description of Embodiments]

Hereinafter, embodiments of the present invention shall be described with reference to the accompanying Drawings.

[Embodiment 1]

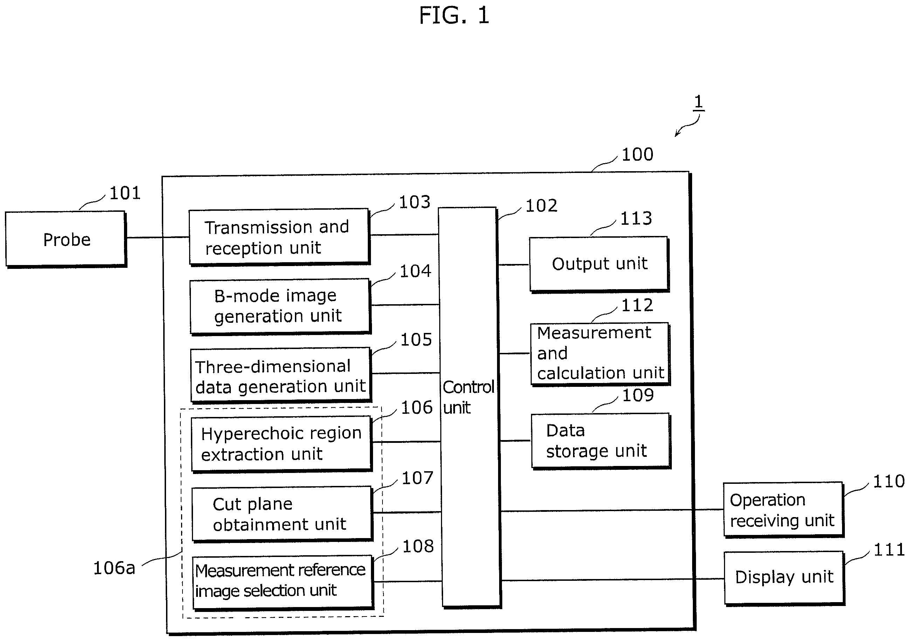

FIG. 1 is a block diagram showing an outline of an ultrasound diagnostic apparatus according to Embodiment 1 of the present invention.

An ultrasound diagnostic apparatus 1 shown in FIG. 1 is configured of an ultrasound diagnostic apparatus main body 100, a probe 101, an operation receiving unit 110, and a display unit 111.

The ultrasound diagnostic apparatus main body 100 includes a control unit 102, a transmission and reception unit 103, a B-mode image generation unit 104, a three-dimensional data generation unit 105, a measurement image selection unit 106a which includes a hyperechoic region extraction unit 106, a cut plane obtainment unit 107, and a measurement reference image selection unit 108, a data storage unit 109, a measurement and calculation unit 112, and an output unit 113.

The probe 101 is connected to the ultrasound diagnostic apparatus main body 100, and ultrasound transducers for transmitting and receiving ultrasound waves are arranged in the probe 101. The probe 101 transmits ultrasound waves according to an instruction from the transmission and reception unit 103, and receives, as echo signals, reflected waves (ultrasound reflected signals) from the body of the subject. The probe 101 also includes a motor which allows the ultrasound transducers to vibrate in a direction that is vertical to a scanning direction. Therefore, when the body of the subject is scanned using the probe 101, the ultrasound transducers scan the body while vibrating, and thus cross-sectional data in the direction vertical to the scanning direction can be obtained based on the echo signals. It should be noted that the probe 101 is not limited to a probe that has a vibration mechanism. For instance, a drive of the ultrasound transducers that are arranged in a matrix in a two-dimensional array probe may be used, or a mechanism which allows the probe 101 to move parallel at a constant speed can also be used. All that is needed for the probe 101 is a means to three-dimensionally transmit and receive the ultrasound waves.

The control unit 102 controls the respective units in the ultrasound diagnostic apparatus main body 100. Note that although it is not specifically stated hereafter, the control unit 102 governs the respective units and operates these units while controlling the operation timings and others.

The transmission and reception unit 103 transmits, to the probe 101, an instruction signal for generating ultrasound waves by driving the ultrasound transducers of the probe 101, and also receives the ultrasound reflected signals from the probe 101.

The B-mode image generation unit 104 generates B-mode images based on the ultrasound reflected signals received by the transmission and reception unit 103. Specifically, the B-mode image generation unit 104 performs, on the ultrasound reflected signals, filtering processing, and then, envelope detection. In addition, the B-mode generation unit 104 performs logarithmic conversion and gain adjustment on the detected signals and outputs the signals that have been converted and adjusted. It should be noted that B-mode is a method to display images by changing the brightness according to the intensity of the ultrasound reflected signals. A B-mode image is a cross-sectional image depicted by changing the intensity of the ultrasound reflected signals into brightness, by changing the ultrasound wave transmission and reception directions in such a way that the probe scans not only in a single scanning direction but scans sequentially along the scanning direction of the probe.

The three-dimensional data generation unit 105 generates three-dimensional data representing an object which is a region in the body of the subject, based on reflected waves reflecting back from the body of the subject after the ultrasound waves have been transmitted towards the body of the subject. Specifically, the three-dimensional data generation unit 105 generates three-dimensional data based on plural B-mode image data generated by the B-mode image generation unit 104. To be more specific, the three-dimensional data generation unit 105 generates three-dimensional data by performing resampling of the pixel values of the B-mode images into three-dimensional coordinate positions. The three-dimensional data generation unit 105 thus reconstitutes the B-mode image data into data that represents the object having a three-dimensional volume, although the details may differ depending on the method used for changing the ultrasonic wave transmitting and receiving directions.

The measurement image selection unit 106a selects, based on the intensity of the reflected waves, one of the two-dimensional cross-sections that compose the three-dimensional data, as a measurement reference image used for measuring a length of the region in the body of the subject. The measurement reference image selection unit 106a includes the hyperechoic region extraction unit 106, the cut plane obtainment unit 107, and the measurement reference image selection unit 108, as has already been mentioned above. The following gives, in more detail, the description of these processing units.

The hyperechoic region extraction unit 106 extracts, from the three-dimensional data, a hyperechoic region which is a region corresponding to the ultrasound reflected signals having a reflection intensity that is greater than a threshold value. Specifically, the hyperechoic region extraction unit 106 extracts only the data that represents such hyperechoic region from the three-dimensional data generated by the three-dimensional data generation unit 105. Here, a hyperechoic region is a region in which the reflection is stronger than the reflections of the neighboring regions whereas a hypoechoic region is a region in which the reflection is weaker than the reflections of the neighboring regions. Thus, with the setting of an appropriate threshold value, the hyperechoic region extraction unit 106 can extract only the data that represents the hyperechoic region, by comparing a three-dimensional data value and the threshold value. In this case, due to the fact that the subject is a fetus, a bone region is mainly extracted as such hyperechoic region.

It should be noted that, in order to prevent the extraction result from being affected by the data condition such as gain variation, it is desirable to firstly obtain a threshold value using a discrimination analysis method, and compare with the threshold value after binarization is performed.

In this manner, the hyperechoic region extraction unit 106 extracts the three-dimensional features of the hyperechoic region (mainly bone region) as a result of extracting, from the three-dimensional data, the data that represents the hyperechoic region.

The cut plane obtainment unit 107 obtains two-dimensional images which compose the three-dimensional data, by cutting the object represented by the three-dimensional data, based on the three-dimensional features of the extracted hyperechoic region. Specifically, the cut plane obtainment unit 107 obtains two-dimensional images (cut planes) by cutting, at a plane, the object represented by the three-dimensional data generated by the three-dimensional data generation unit 105, based on the three-dimensional features of the hyperechoic region extracted by the hyperechoic region extraction unit 106.

More specifically, the cut plane obtainment unit 107 firstly determines an orientation of a cut plane that is a plane at which the object represented by the three-dimensional data is cut based on the three-dimensional features of the hyperechoic region extracted by the hyperechoic region extraction unit 106, and then determines a cutting region which is a region to be cut in the object represented by the three-dimensional data. In other words, the cut plane obtainment unit 107 compares (matches) the three-dimensional data generated by the three-dimensional data generation unit 105 and plural previously-prepared template data which respectively represent the three-dimensional features of the respective specific regions. In the case where the three-dimensional data matches one of the template data, the cut plane obtainment unit 107 determines a three-dimensional region (the object represented by the three-dimensional data) which corresponds to the template data to be the cutting region, and also determines the orientation of the cut plane (the orientation of a surface normal of the cut plane) based on the template data. Then, the cut plane obtainment unit 107 obtains cut planes in the determined cutting region using the determined orientation. In other words, the cut plane obtainment unit 107 obtains the cut planes (two-dimensional images) which have the surface normal of the determined orientation.

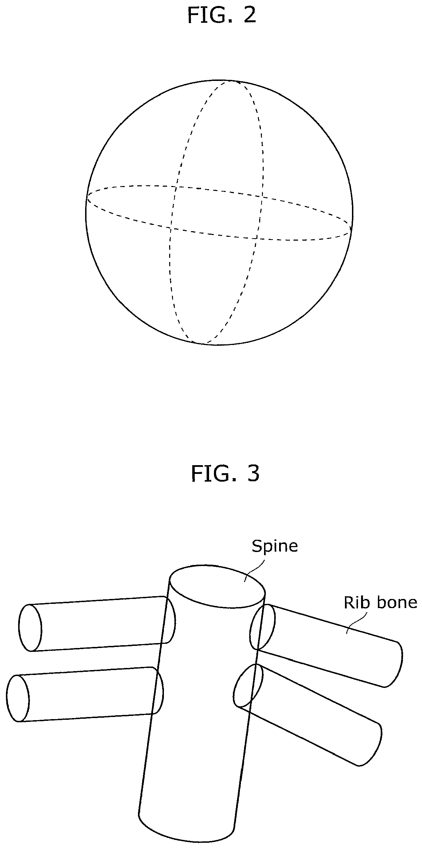

FIG. 2 is a pattern diagram of the previously-prepared template data that represents the three-dimensional features of the head of a fetus. As shown in FIG. 2, the template data representing the head of a fetus is created based on a skull, a dura mater, and a septum pellucidum, and thus represents the locations and the three-dimensional forms of the skull, the dura mater, and the septum pellucidum. The data representing the three-dimensional forms shows that the head is formed in a spherical configuration composed of the skull that has a structure in which curved planes are combined.

Here, it is assumed that the cut plane obtainment unit 107 compares (matches) the three-dimensional data generated by the three-dimensional data generation unit 105 and the respective previously-prepared template data, and the three-dimensional data matches the most with the template data representing the head of a fetus. In such case, the cut plane obtainment unit 107 determines an area that longitudinally traverses the septum pellucidum to be the cutting region, and determines a plane that is vertical to the data representing the septum pellucidum for the orientation of the cut plane. Specifically, in the case where the three-dimensional data matches the most with the template data representing the head of a fetus, the cut plane obtainment unit 107 firstly extracts a median plane of the skull (dura mater) based on the three-dimensional features of the hyperechoic region, and then extracts the septum pellucidum (hypoechoic region) that is longitudinally traversed by the extracted median plane. Then, the cut plane obtainment unit 107 determines the plane that is vertical to the median plane of the skull (dura mater) for the orientation of the cut plane, and determines the area that longitudinally traverses the septum pellucidum (hypoechoic region) to be the cutting region. In this way, the cut plane obtainment unit 107 obtains the cut plane of the head of a fetus based on the bone and the dura mater which are hyperechoic regions.

FIG. 3 is a pattern diagram of the previously-prepared template data representing the three-dimensional features of the abdomen of a fetus. As shown in FIG. 3, the template data representing the abdomen of a fetus is created based on a spine and rib bones, and thus represents the locations and the three-dimensional forms of the spine and the rib bones. The data representing the three-dimensional forms shows that the abdomen is composed of the column-shaped spine which is a collection of bones, and the rib bones which form a symmetrical shape and are made up of bars.

Here, it is assumed that the cut plane obtainment unit 107 compares (matches) the three-dimensional data generated by the three-dimensional data generation unit 105 and the respective previously-prepared template data, and the three-dimensional data matches the most with the template data representing the abdomen of a fetus. In such case, the cut plane obtainment unit 107 determines, for the orientation of the cut plane, a plane that is vertical to the data representing the spine, and determines an area that traverses only the spine to be the cutting region. Specifically, in the case where the three-dimensional data matches the most with the template data representing the abdomen of a fetus, the cut plane obtainment unit 107 firstly extracts a columnar region (hyperechoic region) which is the spine, based on the three-dimensional features of the hyperechoic region. Then, the cut plane obtainment unit 107 determines the plane that is vertical to the extracted columnar region (hyperechoic region) for the orientation of the cut plane, and determines the area that longitudinally traverses only the spine to be the cutting region. In this way, the cut plane obtainment unit 107 obtains the cut plane of the abdomen of a fetus based on the bone which is hyperechoic region.

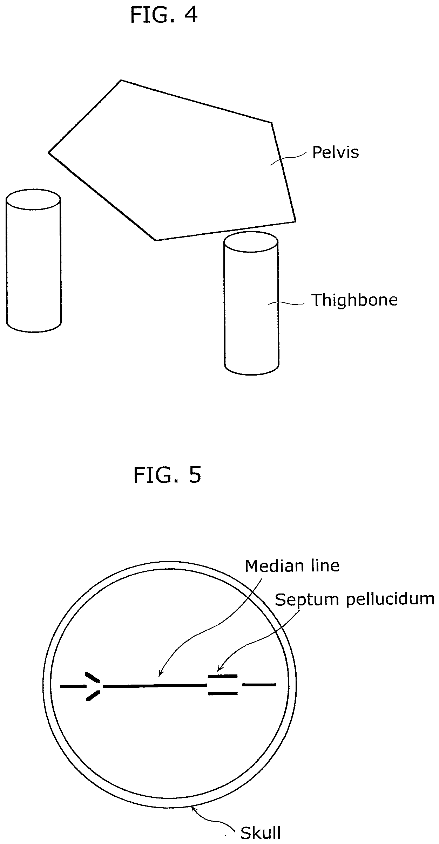

FIG. 4 is a pattern diagram of the previously-prepared template data representing the three-dimensional features of the thigh of a fetus. As shown in FIG. 4, the template data representing the thigh of a fetus is created based on a thighbone and a pelvis, and thus represents the locations and the three-dimensional forms of the thighbone and the pelvis. Specifically, the data representing the three-dimensional forms shows that the thigh is bar-shaped and is joined with a hip joint.

Here, it is assumed that the cut plane obtainment unit 107 compares (matches) the three-dimensional data generated by the three-dimensional data generation unit 105 and the respective previously-prepared template data, and the three-dimensional data matches the most with the template data representing the thigh of a fetus. In such case, the cut plane obtainment unit 107 determines, for the orientation of the cut plane, a plane that traverses the data representing the thighbone, and determines, as the cutting region, an area ranged from 0 to 180 degrees with respect to the data representing the thighbone being located in its center. Specifically, in the case where the three-dimensional data matches the most with the template data representing the thigh of a fetus, the cut plane obtainment unit 107 firstly extracts a bar region (hyperechoic region) which is the thighbone, based on the three-dimensional features of the hyperechoic region. Then, the cut plane obtainment unit 107 determines, for the orientation of the cut plane, the plane that traverses the extracted bar region (hyperechoic region), and determines, as the cutting region, the area having a region that has the plane which traverses the bar region (hyperechoic region) and has the area ranged from 0 to 180 degrees with respect to the determined cut plane. In this way, the cut plane obtainment unit 107 obtains the cut plane of the thigh of a fetus based on the bone which is a hyperechoic region.

As has been described above, the cut plane obtainment unit 107 determines the cutting region and the orientation, and obtains plural cut planes in the determined cutting region using the determined orientation. In other words, the cut plane obtainment unit 107 determines the orientation of two-dimensional image in which the object representing the three-dimensional data is cut, based on the three-dimensional form and location of the extracted hyperechoic region, and thus obtains two-dimensional images in the determined orientation.

The measurement reference image selection unit 108 selects one of the two-dimensional images to be a measurement reference image to be used for measuring a length of a region in the body of the subject. Specifically, the measurement reference image selection unit 108 selects one of the two-dimensional images to be such measurement reference image by evaluating the degree of similarity between each spatial distribution feature of brightness information represented by the respective two-dimensional images and a spatial distribution feature of brightness information represented by the measurement reference image. That is, the measurement reference image selection unit 108 evaluates the cross-sectional images obtained by the cut plane obtainment unit 107, and selects the image that is the most appropriate for measurement to be the measurement reference image. It is desirable to use brightness spatial distribution for the evaluation.

To be more specific, the measurement reference image selection unit 108 studies beforehand a brightness spatial distribution feature that statistically characterizes the measurement reference image, and selects, as such measurement reference image, a cross-sectional image which has a brightness spatial distribution feature that is the closest, among the plural cross-sectional images, to the previously-studied brightness spatial distribution feature of the measurement reference image. In the present embodiment, by comparing the result of the study prepared based on Haar-like features with the result of the feature value calculation performed for the respective cut planes that are obtained by the cut plane obtainment unit 107, the degree of similarity with respect to the measurement reference image can be measured.

The following describes the method for determining measurement reference images for the specific regions that are head, abdomen, and thigh of a fetus which are used for the estimated fetal weight calculation formula.

FIG. 5 is a pattern diagram for describing the features of the measurement cross-section to be used for the measurement of the BPD of a fetus.

In order to accurately measure the BPD (biparietal diameter) of a fetus, it is preferable to measure it using a cross-section of the skull, in which the dura mater and the septum pellucidum are located as shown in FIG. 5. Namely, it is desirable to measure the BPD using the cross-section which is vertical to a median plane of the skull (dura mater) and in which a median line is depicted and the depicted median line traverses the septum pellucidum.

Thus, the measurement reference image selection unit 108 evaluates the cross-sectional images obtained by the cut plane obtainment unit 107, and selects, as a measurement reference image, the measurement cross-section which has the brightness spatial distribution feature that is the most corresponded to the feature shown in FIG. 5. Specifically, the measurement reference image selection unit 108 selects, as a measurement reference image, the cut plane which is vertical to the median plane extracted by the cut plane obtainment unit 107 and in which the median line (hyperechoic region) is depicted in such a way that the extracted hypoechoic region (i.e., septum pellucidum) is traversed.

In this manner, the measurement reference image selection unit 108 selects a measurement reference image based on the bone and the dura mater which are hyperechoic regions.

Note here that the measurement reference image may be a cross-sectional image which shows that the depicted median line further traverses corpora cisterna magna, as shown in FIG. 5.

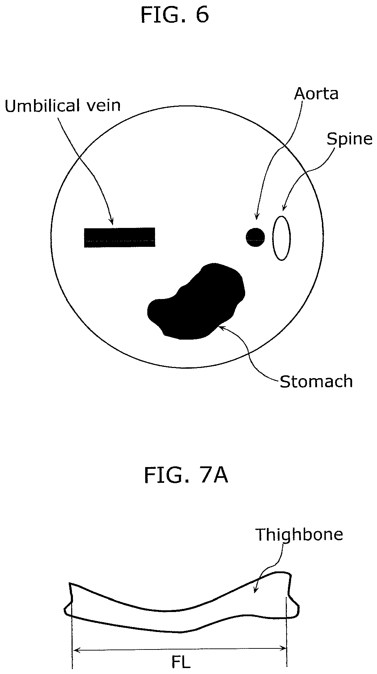

FIG. 6 is a pattern diagram for describing the features of the measurement cross-section to be used for the measurement of the AC of a fetus.

In order to accurately measure the AC (abdominal circumference) of a fetus, it is preferable to measure it using a cross-section of the abdomen, in which the spine, the umbilical vein, and the gastric vesicle are located as shown in FIG. 6. Namely, it is desirable to measure the AC using the cross-section which is almost vertical to the spine (instead of abdominal aorta) and in which the umbilical vein (intrahepatic abdominal umbilical vein) is depicted in the direction almost vertical to the spine and the lumpish gastric vesicle is located near the depicted umbilical vein.

Thus, the measurement reference image selection unit 108 evaluates the cross-sectional images obtained by the cut plane obtainment unit 107, and selects, as a measurement reference image, the measurement cross-section which has the brightness spatial distribution feature that is the most corresponded to the feature shown in FIG. 6. Specifically, the measurement reference image selection unit 108 selects, as a measurement reference image, the cut plane which is vertical to the hyperechoic region (column-shaped region) extracted by the cut plane obtainment unit 107 and in which the hypoechoic region (umbilical vein) is located in the direction almost vertical to the hyperechoic region (column-shaped region) and the lumpish hypoechoic region (gastric vesicle) is located near the hypoechoic region (umbilical vein).

In this manner, the measurement reference image selection unit 108 selects a measurement reference image based on the bone which is hyperechoic region as well as the blood vessels, the stomach and others which are hypoechoic regions.

It should be noted that although it is desirable to select a cut plane based on a spine that can be extracted as a hyperechoic region, a cut plane may be selected based on an abdominal aortic cross that is extracted as a hypoechoic region.

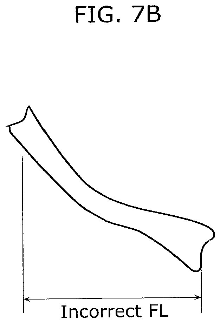

FIG. 7A is a pattern diagram for describing the features of the measurement cross-section to be used for the measurement of the FL of a fetus. FIG. 7B is a diagram schematically showing a measurement cross-section with which the FL of a fetus is measured incorrectly.

In order to accurately measure the FL (femoral length) of a fetus, it is preferable to measure the length of the thighbone as shown in FIG. 7A. Namely, it is desirable to measure the FL using a cross-section that traverses the thighbone.

Thus, the measurement reference image selection unit 108 evaluates the cross-sectional images obtained by the cut plane obtainment unit 107, and selects, as a measurement reference image, the measurement cross-section which has the brightness spatial distribution feature that is the most corresponded to the feature shown in FIG. 7A. Specifically, the measurement reference image selection unit 108 selects, as a measurement reference image, the cut plane that traverses the hyperechoic region (bar-shaped region) extracted by the cut plane obtainment unit 107, that is, the cut plane obtained by cutting the bar-shaped region in the length direction of the bar.

In this manner, the measurement reference image selection unit 108 selects a measurement reference image based on the bone which is a hyperechoic region. As are the other cases, a measurement reference image is determined by evaluating cut planes based on three-dimensional data, not a two-dimensional image (B-mode image). Therefore, it is possible to select, as a measurement reference image, the cross-section with which the length can be accurately measured, as shown in FIG. 7A, not the cross-section with which the length is incorrectly measured, as shown in FIG. 7B.

The data storage unit 109 stores the B-mode images generated by the B-mode image generation unit 104, the three-dimensional data generated by the three-dimensional data generation unit 105, the hyperechoic region data extracted by the hyperechoic region extraction unit 106, and the measurement reference images selected by the measurement reference image selection unit 108.

The operator's instructions are inputted into the operation receiving unit 110. Specifically, the operation receiving unit 110 is configured of buttons, a keyboard, a mouse, and others, and the examiner's instructions are inputted using these.

The display unit 111 is configured of a display device such as an LCD, and displays B-mode images, an object represented by three-dimensional data, and cut planes.

The measurement and calculation unit 112 measures the lengths of the respective regions in the body of the subject using the respectively selected measurement reference images, and calculates an estimated weight of the subject using the lengths that have been measured. Specifically, the measurement and calculation unit 112 measures the lengths of the respective regions in the body of the subject using the measurement reference images respectively selected by the measurement reference image selection unit 108. The measurement and calculation unit 112 then calculates an estimated weight of the subject based on the lengths of the respective regions in the body of the subject which have thus been measured.

The output unit 113 outputs an estimated weight that has been calculated. Specifically, by outputting the estimated weight calculated by the measurement and calculation unit 112, the output unit 113 causes the display unit 111 to display the calculated estimated weight.

The ultrasound diagnostic apparatus 1 according to Embodiment 1 is configured as has been described above.

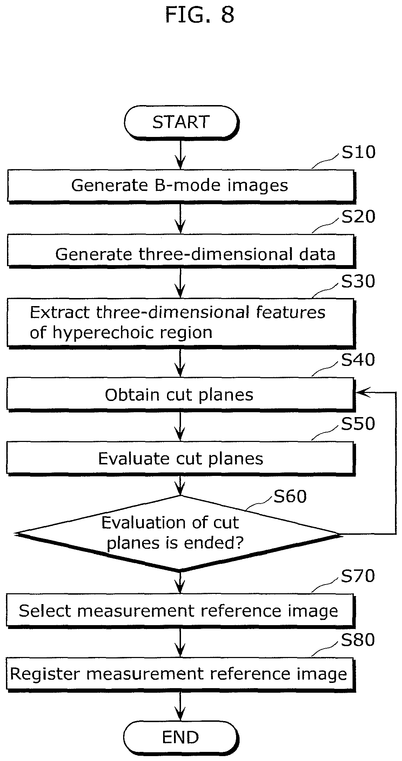

Next, the measurement reference image selection process performed by the ultrasound diagnostic apparatus 1 shall be described with reference to FIG. 8.

FIG. 8 is a flowchart for describing the measurement reference image selection process performed by the ultrasound diagnostic apparatus 1 according to Embodiment 1 of the present invention.

First, the B-mode image generation unit 104 generates B-mode images (step S10).

Specifically, the transmission and reception unit 103 emits ultrasound waves into the body of the subject via the probe 101 and receives the reflected waves via the probe 101. Then, the B-mode image generation unit 104 generates a B-mode image by performing data processing onto the ultrasound reflected signals received by the transmission and reception unit 103, and stores the generated B-mode image into the data storage unit 109. By performing such process while changing the ultrasound wave transmission and reception directions, B-mode images are generated and the generated B-mode images are stored into the data storage unit 109. It should be noted that among the methods of changing the ultrasound wave transmission and reception directions, some use a vibration mechanism of the probe 101, other use a drive of the ultrasound transducers in a two-dimensional array probe, and the others use a mechanism that allows the probe 101 to move parallel at a constant speed, as has already been mentioned above.

Next, the three-dimensional data generation unit 105 generates three-dimensional data based on the B-mode images (step S20). Specifically, the three-dimensional data generation unit 105 generates three-dimensional data by performing resampling of the pixel values of the B-mode images into three-dimensional coordinate positions. The three-dimensional data generation unit 105 thus reconstitutes the B-mode image data into data representing an object that has a three-dimensional volume, although the details may differ depending on the method of changing the ultrasound wave transmission and reception directions.

Then, the hyperechoic region extraction unit 106 extracts a hyperechoic region from the three-dimensional data generated by the three-dimensional data generation unit 105. As a result, the hyperechoic region extraction unit 106 extracts three-dimensional features of the hyperechoic region from the three-dimensional data (step S30).

Then, the cut plane obtainment unit 107 obtains cut planes based on the three-dimensional features of the hyperechoic region (step S40). Specifically, the cut plane obtainment unit 107 compares (matches) the three-dimensional data generated by the three-dimensional data generation unit 105 and each previously-prepared template data which represents the three-dimensional features of the respective specific regions. In the case where the three-dimensional data matches (the degree of similarity is high) one of the template data, the cut plane obtainment unit 107 determines, as the cutting region, the region represented by the three-dimensional data (the object indicated by the three-dimensional data) which corresponds to the template data, and also determines the orientation of a cut plane (the normal orientation of the cut plane) based on the template data. The cut plane obtainment unit 107 then obtains cut planes (two-dimensional images) in the determined cutting region using the determined orientation.

Next, the measurement reference image selection unit 108 evaluates the cut planes obtained by the cut plane obtainment unit 107 (step S50). After having evaluated all the cut planes obtained by the cut plane obtainment unit 107 (step S60), the measurement reference image selection unit 108 then selects, as a measurement reference image, the cut plane that has received the highest evaluation (step S70).

Specifically, by comparing the previously-studied brightness spatial distribution feature which statistically characterizes a measurement reference image and each spatial distribution feature of the respective cut planes obtained by the cut plane obtainment unit 107, the measurement reference image selection unit 108 measures the degree of similarity with respect to the measurement reference image. The measurement reference image selection unit 108 then selects, as a measurement reference image, the cross-sectional image having the brightness spatial distribution feature that is the closest to the previously-studied brightness spatial distribution feature of the measurement reference image, among the cut planes obtained by the cut plane obtainment unit 107.

It should be noted that in the case where the degree of similarity between the feature of the cut plane obtained by the cut plane obtainment unit 107 and the feature of the measurement reference image is low, the measurement reference image selection unit 108 returns to step S40. Then, the cut plane obtainment unit 107 obtains again plural cut planes and proceeds to step S50.

Lastly, the measurement reference image selection unit 108 stores the selected measurement reference image into the data storage unit 109 (step S80).

Thus, the ultrasound diagnostic apparatus 1 performs the measurement reference image selection process. Specifically, the ultrasound diagnostic apparatus 1 determines with accuracy a cross-section that is appropriate for measurement, by narrowing down the number of cut planes based on the three-dimensional features of the bone region that is to be a hyperechoic region, for the obtainment of an appropriate cut plane.

It should be noted that, in step S30, the examiner may judge on the region in the body of the subject based on the three-dimensional features of the hyperechoic region (three-dimensional form and location information of the hyperechoic region) extracted by the hyperechoic region extraction unit 106. In such case, the examiner may notify, via the operation receiving unit 110, the cut plane obtainment unit 107 that the three-dimensional data generated by the three-dimensional data generation unit 105 is the data representing a specific region such as a thigh, for instance, and may thus select down in advance the template data which represents such a specific region and is to be compared (matched) with the three-dimensional data generated by the three-dimensional data generation unit 105. In this way, it is possible to improve the efficiency in the process performed by the cut plane obtainment unit 107 in step S40. In addition, it is also possible to improve the efficiency in the evaluation performed by the measurement reference image selection unit 108 in step S50, and thus to reduce the risk of false evaluation.

Thus, the ultrasound diagnostic apparatus 1 performs the measurement reference image selection process. This enables those who are not skillful in operating an ultrasound diagnostic apparatus to surely obtain an appropriate measurement reference image, and to accurately measure the length of a specific region based on such measurement reference image.

The following shall describe the whole processing performed by the ultrasound diagnostic apparatus 1, that is, the processing which includes the measurement reference image selection process and is up to the process in which the ultrasound diagnostic apparatus 1 calculates an estimated weight of the subject.

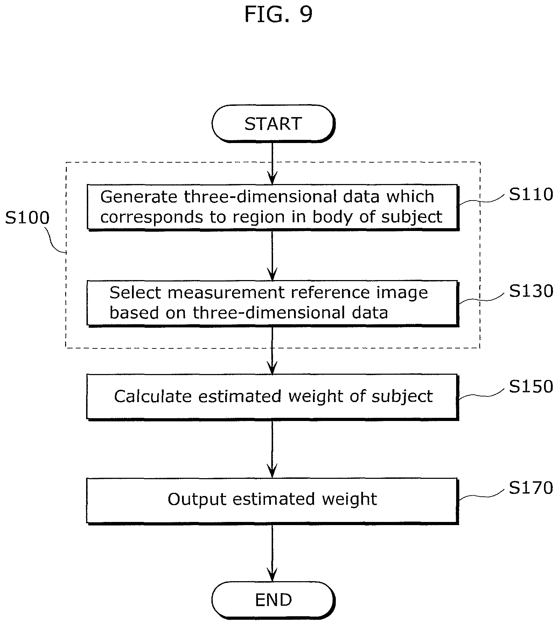

FIG. 9 is a flowchart for describing the processing that is up to the process of calculating an estimated weight of the subject, which is performed by the ultrasound diagnostic apparatus according to Embodiment 1 of the present invention.

The ultrasound diagnostic apparatus 1 firstly generates three-dimensional data for a region in the body of the subject based on the reflected waves of the ultrasound waves which have been transmitted towards the body of the subject and reflected back from the body of the subject. (S110). Specifically, the ultrasound diagnostic apparatus 1 performs the processing in steps S10 and S20 described in FIG. 8. Since the processing in steps S10 and S20 has been described above, the description thereof shall not be repeated here.

Then, the ultrasound diagnostic apparatus 1 selects, based on the intensity of the reflected waves from the body of the subject, one of the two-dimensional images that compose the three-dimensional data, as a measurement reference image to be used for measuring a length of the region in the body of the subject (S130). Specifically, the ultrasound diagnostic apparatus 1 performs the processing from steps S30 to S80 described in FIG. 8. Since the processing from steps S30 to S80 has already been described above, the description thereof shall not be repeated here.

It should be noted that, in steps S110 and S130, more precisely, the three-dimensional data is generated for the respective regions in the body of the subject, namely, the head, abdomen, and thigh of a fetus.

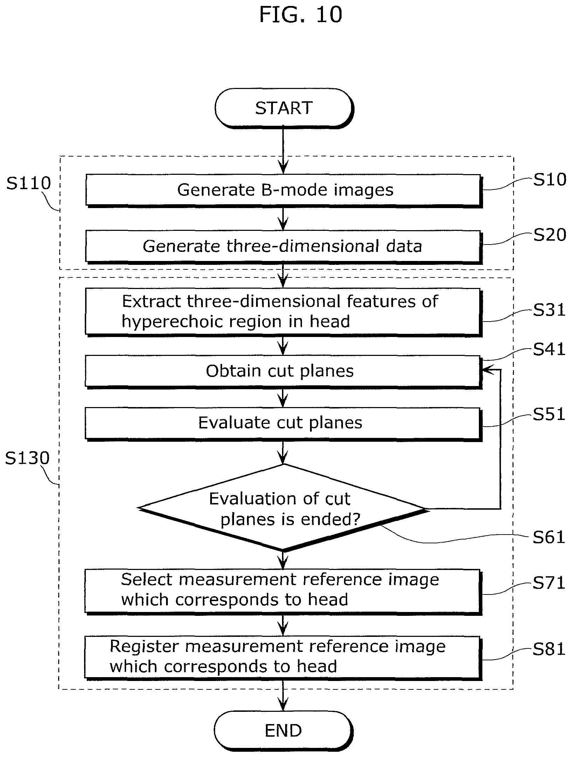

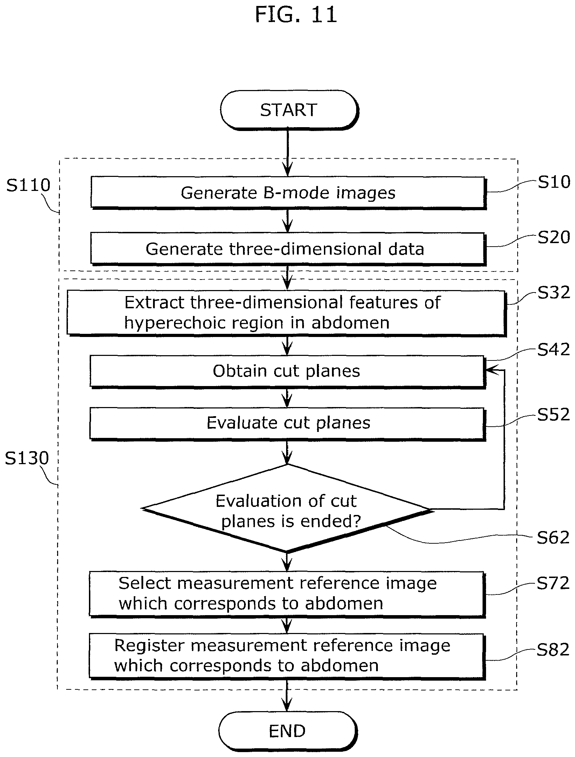

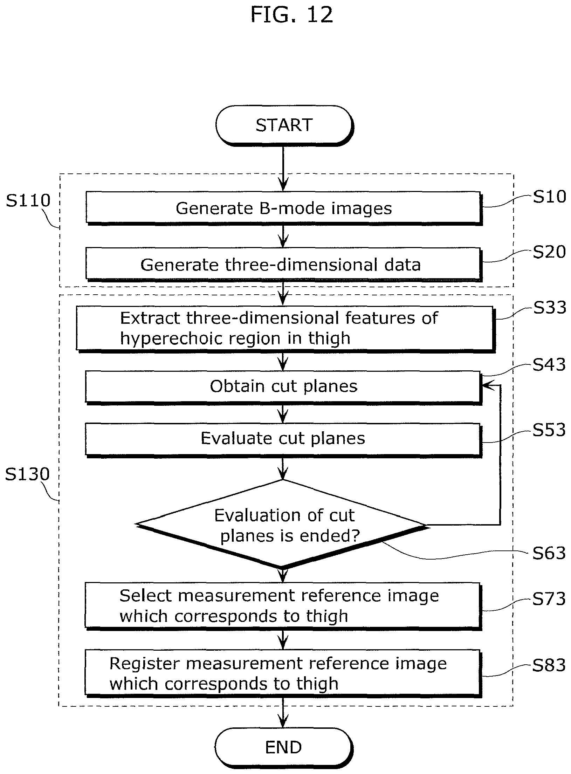

FIG. 10 is a flowchart showing the measurement reference image selection process performed for the head of a fetus, according to Embodiment 1 of the present invention. FIG. 11 is a flowchart showing the measurement reference image selection process performed for the abdomen of a fetus, according to Embodiment 1. FIG. 12 is a flowchart showing the measurement reference image selection process performed for the thigh of a fetus, according to Embodiment 1. The constituent elements that are the same as those described in FIG. 8 use the same reference numerals, and the description thereof shall not be repeated.

As shown in FIG. 10, in the case where the three-dimensional data generated in step S110 corresponds to the head of a fetus, the three-dimensional features of the hyperechoic region in the head are extracted in step S31. After that, a measurement reference image to be used for measuring a length of the head of a fetus is selected in step S71, and the selected measurement reference image is registered in step S81. It should be noted that the processing from steps S31 to S81 corresponds to the processing from steps S30 to S80 described in FIG. 8; therefore, the description thereof shall not be repeated. In addition, as shown in FIG. 11, in the case where the three-dimensional data generated in step S110 corresponds to the abdomen of a fetus, the three-dimensional features of the hyperechoic region in the abdomen are extracted in step S32. After that, a measurement reference image to be used for measuring a length of the abdomen of a fetus is selected in step S72, and the selected measurement reference image is registered in step S82. It should be noted that the processing from steps S32 to S82 corresponds to the processing from steps S30 to S80 described in FIG. 8; therefore, the description thereof shall not be repeated. Furthermore, as shown in FIG. 12, in the case where the three-dimensional data generated in step S110 corresponds to the thigh of a fetus, the three-dimensional features of the hyperechoic region in the thigh are extracted in step S33. After that, a measurement reference image to be used for measuring a length of the thigh of a fetus is selected in step S73, and the selected measurement reference image is registered in step S83. It should be noted that the processing from steps S33 to S83 corresponds to the processing from steps S30 to S80 described in FIG. 8; therefore, the description thereof shall not be repeated.

Next, the ultrasound diagnostic apparatus 1 measures the lengths of the respective regions in the body of the subject using the measurement reference images respectively selected in S130, and calculates an estimated weight of the subject based on the measured lengths (S150).

Specifically, the measurement and calculation unit 112 measures the lengths of the respective regions in the body of the subject using the respectively selected measurement reference images, and calculates an estimated weight of the subject using the measured lengths.

Then, the ultrasound diagnostic apparatus 1 outputs the calculated estimated weight (S170).

Thus, the ultrasound diagnostic apparatus 1 calculates an estimated weight of the subject.

According to the present embodiment, it is possible to achieve the ultrasound diagnostic apparatus capable of reducing the dependence on the examiner and calculating an estimated fetal weight with high accuracy and easy operation.

[Embodiment 2]

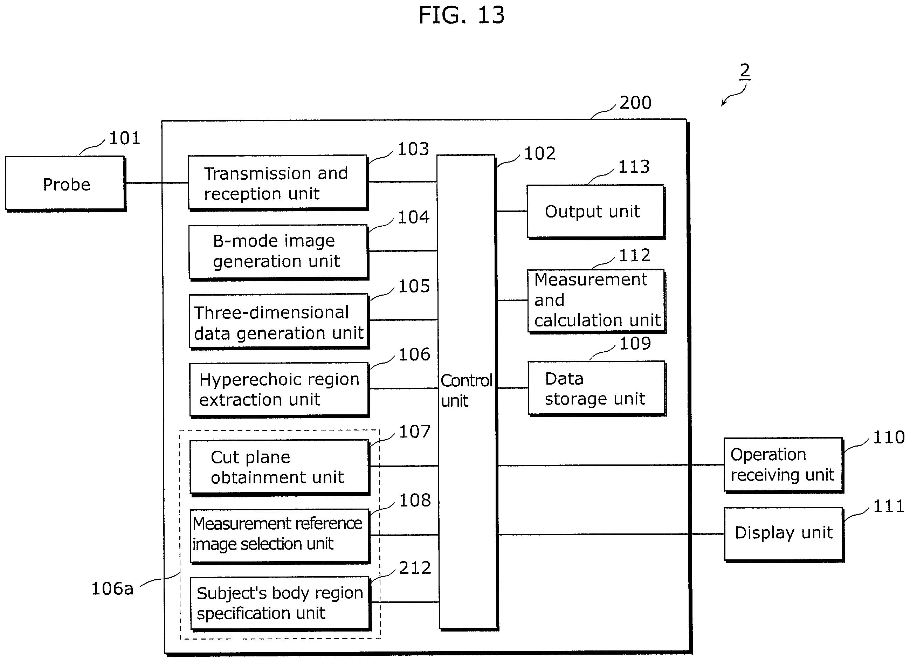

FIG. 13 is a block diagram showing an outline of the ultrasound diagnostic apparatus according to Embodiment 2 of the present invention. In FIG. 13, constituent elements that are the same as those in FIG. 1 use the same reference numerals, and the description thereof shall not be repeated.

The ultrasound diagnostic apparatus 2 shown in FIG. 13 is configured of an ultrasound diagnostic apparatus main body 200, the probe 101, the operation receiving unit 110, and the display unit 111. The configuration of a subject's body region specification unit 212 is what makes the ultrasound diagnostic apparatus main body 200 shown in FIG. 13 different from the ultrasound diagnostic apparatus main body 100 shown in FIG. 1. Namely, the ultrasound diagnostic apparatus main body 200 has the subject's body region specification unit 212 in addition to the configuration shown in FIG. 1.

The subject's body region specification unit 212 specifies a region, in the body of the subject, which is the object represented by the three-dimensional data. Specifically, the subject's body region specification unit 212 judges that the object represented by the three-dimensional data generated by the three-dimensional data generation unit 105 is a region, for instance, a head, an abdomen, or a thigh. The judgment is based on the three-dimensional features of the hyperechoic region (three-dimensional form and location information of the hyperechoic region) extracted by the hyperechoic region extraction unit 106. The subject's body region specification unit 212 thus specifies the region in the body of the subject (three-dimensional data) which is being observed.

For example, the subject's body region specification unit 212 compares the three-dimensional data generated by the three-dimensional data generation unit 105 and the template data (e.g., FIG. 2) which represents the head of a fetus and has predefined features of a skull. In the case where both data have similar features (resemble), the subject's body region specification unit 212 judges that the object represented by the three-dimensional data is a head. In addition, the subject's body region specification unit 212 compares the three-dimensional data generated by the three-dimensional data generation unit 105 and the template data (e.g., FIG. 3) which represents the abdomen of a fetus and has predefined features of a spine. In the case where both data have similar features (resemble), the subject's body region specification unit 212 judges that the object represented by the three-dimensional data is an abdomen. Likewise, the subject's body region specification unit 212 compares the three-dimensional data generated by the three-dimensional data generation unit 105 and the template data (e.g., FIG. 4) which represents the thigh of a fetus and has predefined features of a thighbone. In the case where both data have similar features (resemble), the subject's body region specification unit 212 judges that the object represented by the three-dimensional data is a thigh.

The ultrasound diagnostic apparatus 2 according to Embodiment 2 is configured as has been described above.

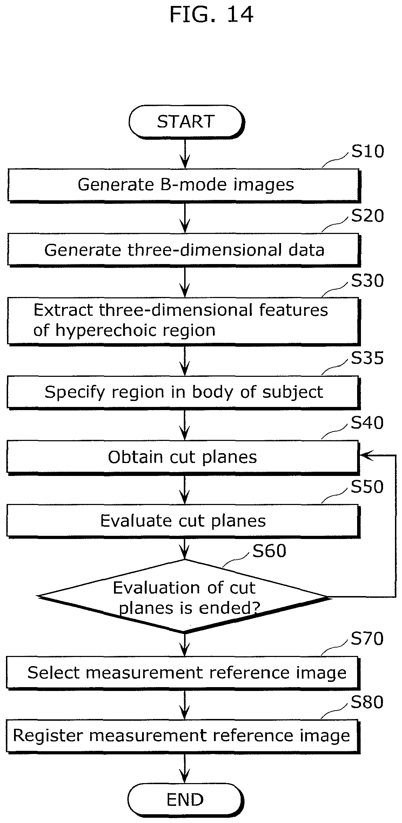

FIG. 14 is a flowchart for describing the measurement reference image selection process performed by the ultrasound diagnostic apparatus according to Embodiment 2 of the present invention. The constituent elements that are the same as those in FIG. 8 use the same reference numerals, and the description thereof shall not be repeated.

The difference between FIG. 14 and FIG. 8 is that step S35 is added.

In step S35, the subject's body region specification unit 212 judges that the object represented by the three-dimensional data generated by the three-dimensional data generation unit 105 is a region, for instance, a head, an abdomen, or a thigh. The judgment is based on the three-dimensional features of the hyperechoic region (three-dimensional form and location information of the hyperechoic region) extracted by the hyperechoic region extraction unit 106. The subject's body region specification unit 212 thus specifies the region in the body of the subject (three-dimensional data) which is being observed.

Next, the ultrasound diagnostic apparatus 2 proceeds to step S40, and the cut plane obtainment unit 107 obtains two-dimensional images based on the information indicating the three-dimensional form and location of the region specified by the subject's body region specification unit 212 and the three-dimensional form and location of the extracted hyperechoic region.

For example, in the case where the subject's body region specification unit 212 specifies that the region of a fetus, which is the object represented by the three-dimensional data, is a head, the cut plane obtainment unit 107 extracts a region that corresponds to a septum pellucidum, based on the three-dimensional features of the extracted hyperechoic region, determines, based on the extracted region, the orientation of two-dimensional image in which the object represented by the three-dimensional data is cut, and obtains two-dimensional images in the determined orientation.

In addition, in the case where the subject's body region specification unit 212 specifies that the region of a fetus, which is the object represented by the three-dimensional data, is an abdomen, the cut plane obtainment unit 107 extracts a region that corresponds to a spine, based on the three-dimensional features of the extracted hyperechoic region, determines, based on the extracted region, the orientation of two-dimensional image in which the object represented by the three-dimensional data is cut, and obtains two-dimensional images in the determined orientation.

Furthermore, in the case where the subject's body region specification unit 212 specifies that the region of a fetus, which is the object represented by the three-dimensional data, is a thigh, the cut plane obtainment unit 107 extracts a region that corresponds to a thighbone, based on the three-dimensional features of the extracted hyperechoic region, determines, based on the extracted region, the orientation of two-dimensional image in which the object represented by the three-dimensional data is cut, and obtains two-dimensional images in the determined orientation.

Thus, the ultrasound diagnostic apparatus 2 performs the measurement reference image selection process.

As described above, the ultrasound diagnostic apparatus 2 according to the present embodiment thus performs efficient evaluation and reduces the risk of false evaluation. With this, the ultrasound diagnostic apparatus 2 can further select, with high accuracy, a cross-section (measurement reference image) that is appropriate for measurement.

It should be noted that, in the present embodiment, the subject's body region specification unit 212 is configured to judge based on the features of a hyperechoic region, however, the examiner may give an instruction via the operation receiving unit 110. In other words, the subject's body region specification unit 212 may specify a region, in the body of the subject, which is the object represented by the three-dimensional data, according to the examiner's (operator's) instruction received by the operation receiving unit 110. In such case, although such examiner's instruction is a step added to the process, a region in the body of the subject can be precisely determined, which enables more stable obtainment of the measurement reference image that is appropriate for measurement.

According to the present invention, it is possible to realize the ultrasound diagnostic apparatus capable of reducing the dependence on the examiner and calculating an estimated fetal weight with high accuracy and easy operation.

It should be noted that although it has been described in the embodiments that the probe 101 and the ultrasound diagnostic apparatus 100 are separately configured, the present invention is not limited to these embodiments. The probe 101 may include part or all of the processing units included in the ultrasound diagnostic apparatus main body 100.

In the above description, the ultrasound diagnostic apparatus main body 100 includes the control unit 102, the transmission and reception unit 103, the B-mode image generation unit 104, the three-dimensional data generation unit 105, the hyperechoic region extraction unit 106, the measurement image selection unit 106a, the data storage unit 109, the measurement and calculation unit 112, and the output unit 113. However, the present invention is not limited to such configuration. As shown in FIG. 15, the ultrasound diagnostic apparatus main body 100 may include a minimum configuration 100a as a minimum configuration. Namely, the ultrasound diagnostic apparatus main body 100 may include the three-dimensional data generation unit 105, the measurement image selection unit 106a, the measurement and calculation unit 112, the output unit 113 and the control unit 102. FIG. 15 is a diagram showing the minimum configuration of the ultrasound diagnostic apparatus according to the present invention.

With the configuration of the ultrasound diagnostic apparatus 1 which includes at least such minimum configuration 100a, it is possible to realize the ultrasound diagnostic apparatus capable of reducing the dependence on the examiner and calculating an estimated fetal weight with high accuracy and easy operation.

Furthermore, in the above description, the measurement and calculation unit 112 performs measurements using the measurement reference images determined by the measurement reference image selection unit 108, and calculates an estimated weight of a fetus being the subject, based on the measured lengths of the regions in the body of the subject. However, the present invention is not limited to this. The ultrasound diagnostic apparatus main body 100 may include neither the measurement and calculation unit 112 nor the output unit 113, and the examiner may calculate an estimated fetal weight based on the lengths of the regions in the body of the subject, which have been measured using the measurement reference images determined by the measurement reference image selection unit 108.

Although the ultrasound diagnostic apparatuses according to the embodiments of the present invention have been described up to this point, the present invention is not limited to these embodiments. As long as they do not depart from the essence of the present invention, various modifications obtainable through modifications to the respective embodiments that may be conceived by a person of ordinary skill in the art as well as an embodiment composed by the combination of the constituent elements of different embodiments are intended to be included in the scope of the present invention.

For example, the present invention may be the method as described herein, or a computer program for achieving such method by a computer, or a digital signal composed of such computer program.

Furthermore, the present invention may be the aforementioned computer program or digital signal which is recorded in a computer-readable recording medium, such as a flexible disc, a hard disc, a CD-ROM, an MO, a DVD, a DVD-ROM, a DVD-RAM, a BD (Blu-Ray Disc), a semiconductor memory or the like. The present invention may also be the digital signal recorded in such recording medium.

Furthermore, according to the present invention, the aforementioned computer program or digital signal may be transferred via an electric communication line, a wireless or wired communication line, or a network as represented by the Internet, a data broadcasting, etc.

The present invention may be a computer system comprised of a microprocessor and a memory, in which the memory stores the aforementioned computer program and the microprocessor is operated according to such computer program.

The present invention may be implemented in other independent computer system by transferring the aforementioned program or digital signal which has been recorded in the aforementioned recording medium, or by transferring such program or digital signal via the aforementioned network.

[Industrial Applicability]

The present invention is applicable to ultrasound diagnostic apparatuses, and can be applied, in particular, to an ultrasound diagnostic apparatus capable of easily and properly obtaining measurement reference images for the thorough examination on the growth of a fetus.

[Reference Signs List]

| 1, 2 | ultrasound diagnostic apparatus |
| 100, 200 | ultrasound diagnostic apparatus main body |
| 101 | probe |
| 102 | control unit |

| 103 | transmission and reception unit |
| 104 | B mode image generation unit |
| 105 | three-dimensional data generation unit |
| 106 | hyperechoic region extraction unit |
| 106a | measurement image selection unit |
| 107 | cut plane obtainment unit |
| 108 | measurement reference image selection unit |
| 109 | data storage unit |
| 110 | operation receiving unit |
| 111 | display unit |
| 112 | measurement and calculation unit |
| 113 | output unit |
| 212 | subject's body region specification unit |

**Claims**

1. An ultrasound diagnostic apparatus (1, 2) comprising:

   a three-dimensional data generation unit (105) configured to generate three-dimensional data for one or more regions in a body of a subject based on reflected waves reflecting back from the body of the subject after ultrasound waves have been transmitted towards the body of the subject;
   a measurement image selection unit (106a) configured to select, based on an intensity of the reflected waves, one of two-dimensional cross-sections that compose the three-dimensional data, as a measurement reference image used for measuring a length of each region in the body of the subject;
   a measurement and calculation unit (112) configured to measure the length of each region in the body of the subject using the selected measurement reference image, and to calculate an estimated weight of the subject using the measured lengths; and
   an output unit (113) configured to output the calculated estimated weight,

   **characterised in that**
   said measurement image selection unit (106a) includes:

   a hyperechoic region extraction unit (106) configured to extract, from the three-dimensional data, a hyperechoic region which is a region corresponding to the reflected waves having a reflection intensity that is greater than a threshold value;
   a cut plane obtainment unit (107) configured to obtain two-dimensional cross-sections that compose the three-dimensional data, by cutting the three-dimensional data based on a three-dimensional feature of the extracted hyperechoic region; and
   a reference image selection unit (108) configured.to select one of the two-dimensional cross-sections as the measurement reference image used for measuring the length of the region in the body of the subject.

2. The ultrasound diagnostic apparatus (1, 2) according to Claim 1,
   wherein said cut plane obtainment unit (107) is configured to determine, based on three-dimensional form and location of the extracted hyperechoic region, an orientation of two-dimensional cross-section in which the three-dimensional data is cut, and to obtain two-dimensional cross-sections in the determined orientation.

3. The ultrasound diagnostic apparatus (2) according to Claim 1 or Claim 2, further comprising:

   a subject's body region specification unit (212) configured to specify the region, in the body of the subject, which corresponds to the three-dimensional data,
   wherein said cut plane obtainment unit (107) is configured to obtain two-dimensional cross-sections based on information indicating three-dimensional form and location of the region specified by said subject's body region specification unit and also based on the three-dimensional form and location of the extracted hyperechoic region.

4. The ultrasound diagnostic apparatus (2) according to Claim 3,
   wherein said subject's body region specification unit (212) is configured to specify that the region in the body of the subject is at least one of head, abdomen, and thigh, the region corresponding to the three-dimensional data.

5. The ultrasound diagnostic apparatus (2) according to Claim 4, further comprising:

an operation receiving unit (110) configured to receive an instruction from an operator,
wherein said subject's body region specification unit (212) is configured to specify the region in the body of the subject according to the instruction from the operator received by said operation receiving unit, the region corresponding to the three-dimensional data.

6. The ultrasound diagnostic apparatus (2) according to Claim 4,
wherein said subject's body region specification unit (212) is configured to specify the region in the body of the subject based on the three-dimensional form of the extracted hyperechoic region, the region corresponding to the three-dimensional data.

7. The ultrasound diagnostic apparatus (2) according to Claim 5 or Claim 6,
wherein in the case where said subject's body region specification unit (212) specifies that the region, in the body of the subject, which corresponds to the three-dimensional data, is head, said cut plane obtainment unit (107) is configured to: extract a region of a septum pellucidum based on the three-dimensional features of the hyperechoic region; determine, based on the extracted region, an orientation of two-dimensional cross-section in which the three-dimensional data is cut; and obtain two-dimensional cross-sections in the determined orientation.

8. The ultrasound diagnostic apparatus (2) according to Claim 5 or Claim 6,
wherein in the case where said subject's body region specification unit (212) specifies that the region, in the body of the subject, which corresponds to the three-dimensional data, is abdomen, said cut plane obtainment unit (107) is configured to: extract a region of a spine based on the three-dimensional features of the hyperechoic region; determine, based on the extracted region, an orientation of two-dimensional cross-section in which the three-dimensional data is cut; and obtain two-dimensional cross-sections in the determined orientation.

9. The ultrasound diagnostic apparatus (2) according to Claim 5 or Claim 6,
wherein in the case where said subject's body region specification unit (212) specifies that the region, in the body of the subject, which corresponds to the three-dimensional data, is thigh, said cut plane obtainment unit (107) is configured to: extract a region of a thighbone based on the three-dimensional features of the hyperechoic region; determine, based on the extracted region, an orientation of two-dimensional cross-section in which the three-dimensional data is cut; and obtain two-dimensional cross-sections in the determined orientation.

10. The ultrasound diagnostic apparatus (1, 2) according to Claim 1,
wherein said reference image selection unit (108) is configured to select one of the two-dimensional cross-sections as the measurement reference image by evaluating a degree of similarity between a spatial distribution feature of brightness information represented by each of the two-dimensional cross-sections and a spatial distribution feature of brightness information represented by the measurement reference image.

11. An image processing method comprising:

generating (S20, S110) three-dimensional data for a region in a body of a subject, based on reflected waves reflecting back from the body of the subject after ultrasound waves have been transmitted towards the body of the subject;
selecting (S70, S130), based on an intensity of the reflected waves, one of two-dimensional cross-sections that compose the three-dimensional data, as a measurement reference image used for measuring a length of each region in the body of the subject;
measuring the length of each region in the body of the subject using the selected measurement reference image, and calculating (S150) an estimated weight of the subject using the measured lengths; and
outputting (S170) the calculated estimated weight,

**characterised in that**
said selecting includes:

extracting (S30), from the three-dimensional data, a hyperechoic region which is a region corresponding to the reflected waves having a reflection intensity that is greater than a threshold value;
obtaining (S40) two-dimensional cross-sections that compose the three-dimensional data, by cutting the three-dimensional data based on a three-dimensional feature of the extracted hyperechoic region; and

selecting (S70) one of the two-dimensional cross-sections as the measurement reference image used for measuring the length of the region in the body of the subject.

**Patentansprüche**

1. Ultraschalldiagnosevorrichtung (1, 2), aufweisend:

   eine Dreidimensionale-Daten-Erzeugungseinheit (105), die konfiguriert ist, um dreidimensionale Daten für ein oder mehrere Gebiete in dem Körper einer Testperson basierend auf reflektierten Wellen, die von dem Körper der Testperson zurück reflektieren, nachdem Ultraschallwellen zu dem Körper der Testperson übertragen worden sind, zu erzeugen,
   eine Messbild-Auswahleinheit (106a), die konfiguriert ist, um basierend auf einer Intensität der reflektierten Wellen einen von zweidimensionalen Querschnitten, welche die dreidimensionalen Daten bilden, als ein Messbezugsbild, das zum Messen einer Länge von jedem Gebiet in dem Körper der Testperson verwendet wird, auszuwählen,
   eine Mess- und Berechnungseinheit (112), die konfiguriert ist, um die Länge von jedem Gebiet in dem Körper der Testperson unter Verwendung des ausgewählten Messbezugsbildes zu messen, und um ein geschätztes Gewicht der Testperson unter Verwendung der gemessenen Längen zu berechnen, und
   eine Ausgabeeinheit (113), die konfiguriert ist, um das berechnete, geschätzte Gewicht auszugeben,

   **dadurch gekennzeichnet, dass**
   die Messbild-Auswahleinheit (106a) umfasst:

   eine Echoreiches-Gebiet-Extraktionseinheit (106), die konfiguriert ist, um aus den dreidimensionalen Daten ein echoreiches Gebiet, welches ein Gebiet korrespondierend zu den reflektierten Wellen mit einer Reflexionsintensität größer als ein Schwellenwert ist, zu extrahieren,
   eine Schnittebene-Erhaltungseinheit (107), die konfiguriert ist, um zweidimensionale Querschnitte, welche die dreidimensionalen Daten bilden, zu erhalten, indem die dreidimensionalen Daten basierend auf einem dreidimensionalen Merkmal des extrahierten echoreichen Gebiets geschnitten werden, und
   eine Bezugsbild-Auswahleinheit (108), die konfiguriert ist, um einen der zweidimensionalen Querschnitte als das Messbezugsbild, das zum Messen der Länge des Gebiets in dem Körper der Testperson verwendet wird, auszuwählen.

2. Ultraschalldiagnosevorrichtung (1, 2) nach Anspruch 1,
   wobei die Schnittebene-Erhaltungseinheit (107) konfiguriert ist, um basierend auf einer dreidimensionalen Form und Position des extrahierten echoreichen Gebiets eine Orientierung eines zweidimensionalen Querschnitts, in welchem die dreidimensionalen Daten geschnitten sind, zu bestimmen, und um zweidimensionale Querschnitte in der bestimmten Orientierung zu erhalten.

3. Ultraschalldiagnosevorrichtung (2) nach Anspruch 1 oder 2, ferner aufweisend:

   eine Testperson-Körpergebiet-Spezifikationseinheit (212), die konfiguriert ist, um das Gebiet in dem Körper der Testperson, welches zu den dreidimensionalen Daten korrespondiert, zu spezifizieren,
   wobei die Schnittebene-Erhaltungseinheit (107) konfiguriert ist, um zweidimensionale Querschnitte basierend auf Informationen, die eine dreidimensionale Form anzeigen, und einer Position des durch die Testperson-Körpergebiet-Spezifikationseinheit spezifizierten Gebiets, und auch basierend auf der dreidimensionalen Form und der Position des extrahierten echoreichen Gebiets zu erhalten.

4. Ultraschalldiagnosevorrichtung (2) nach Anspruch 3, wobei die Testperson-Körpergebiet-Spezifikationseinheit (212) konfiguriert ist, um zu spezifizieren, dass das Gebiet in dem Körper der Testperson zumindest eines des Kopfes, des Abdomens und des Oberschenkels ist, wobei das Gebiet zu den dreidimensionalen Daten korrespondiert.

5. Ultraschalldiagnosevorrichtung (2) nach Anspruch 4, ferner aufweisend:

   eine Betriebsempfangseinheit (110), die konfiguriert ist, um eine Anweisung von einem Bediener zu empfangen,
   wobei die Testperson-Körpergebiet-Spezifikationseinheit (212) konfiguriert ist, um das Gebiet in dem Körper der Testperson gemäß der durch die Betriebsempfangseinheit empfangenen Anweisung von dem Bediener zu spezifizieren, wobei das Gebiet zu den dreidimensionalen Daten korrespondiert.

6. Ultraschalldiagnosevorrichtung (2) nach Anspruch 4, wobei die Testperson-Körpergebiet-Spezifikationseinheit (212) konfiguriert ist, um das Gebiet in dem Körper der Testperson basierend auf der dreidimensionalen Form des extrahierten echoreichen Gebiets zu spezifizieren, wobei das Gebiet zu den dreidimensionalen Daten korrespondiert.

7. Ultraschalldiagnosevorrichtung (2) nach Anspruch 5 oder 6,
wobei in dem Fall, dass die Testperson-Körpergebiet-Spezifikationseinheit (212) spezifiziert, dass das Gebiet in dem Körper der Testperson, welches zu den dreidimensionalen Daten korrespondiert, der Kopf ist, die Schnittebene-Erhaltungseinheit (107) konfiguriert ist, um ein Gebiet eines Septum pellucidum basierend auf den dreidimensionalen Merkmalen des echoreichen Gebiets zu extrahieren; um basierend auf dem extrahierten Gebiet eine Orientierung eines zweidimensionalen Querschnitts, in welchem die dreidimensionalen Daten geschnitten sind, zu bestimmen; und um zweidimensionale Querschnitte in der bestimmten Orientierung zu erhalten.

8. Ultraschalldiagnosevorrichtung (2) nach Anspruch 5 oder 6,
wobei in dem Fall, dass die Testperson-Körpergebiet-Spezifikationseinheit (212) spezifiziert, dass das Gebiet in dem Körper der Testperson, welches zu den dreidimensionalen Daten korrespondiert, der Abdomen ist, die Schnittebene-Erhaltungseinheit (107) konfiguriert ist, um ein Gebiet einer Wirbelsäule basierend auf den dreidimensionalen Merkmalen des echoreichen Gebiets zu extrahieren; um basierend auf dem extrahierten Gebiet eine Orientierung eines zweidimensionalen Querschnitts, in welchem die dreidimensionalen Daten geschnitten sind, zu bestimmen; und um zweidimensionale Querschnitte in der bestimmten Orientierung zu erhalten.

9. Ultraschalldiagnosevorrichtung (2) nach Anspruch 5 oder 6,
wobei in dem Fall, dass die Testperson-Körpergebiet-Spezifikationseinheit (212) spezifiziert, dass das Gebiet in dem Körper der Testperson, welches zu den dreidimensionalen Daten korrespondiert, der Oberschenkel ist, die Schnittebene-Erhaltungseinheit (107) konfiguriert ist, um ein Gebiet eines Oberschenkelknochens basierend auf den dreidimensionalen Merkmalen des echoreichen Gebiets zu extrahieren; um basierend auf dem extrahierten Gebiet eine Orientierung eines zweidimensionalen Querschnitts, in welchem die dreidimensionalen Daten geschnitten sind, zu bestimmen; und um zweidimensionale Querschnitte in der bestimmten Orientierung zu erhalten.

10. Ultraschalldiagnosevorrichtung (1, 2) nach Anspruch 1,
wobei die Bezugsbild-Auswahleinheit (108) konfiguriert ist, um einen der zweidimensionalen Querschnitte als das Messbezugsbild auszuwählen, indem ein Ähnlichkeitsgrad zwischen einem räumlichen Verteilungsmerkmal von Helligkeitsinformationen, die durch jeden der zweidimensionalen Querschnitte dargestellt sind, und einem räumlichen Verteilungsmerkmal von Helligkeitsinformationen, die durch das Messbezugsbild dargestellt sind, bewertet wird.

11. Bildverarbeitungsverfahren, aufweisend:

Erzeugen (S20, S110) von dreidimensionalen Daten für ein Gebiet in dem Körper einer Testperson basierend auf reflektierten Wellen, die von dem Körper der Testperson zurück reflektieren, nachdem Ultraschallwellen zu dem Körper der Testperson übertragen worden sind,
Auswählen (S70, S130) basierend auf einer Intensität der reflektierten Wellen eines von zweidimensionalen Querschnitten, welche die dreidimensionalen Daten bilden, als ein Messbezugsbild, das zum Messen einer Länge von jedem Gebiet in dem Körper der Testperson verwendet wird,
Messen der Länge von jedem Gebiet in dem Körper der Testperson unter Verwendung des ausgewählten Messbezugsbildes, und Berechnen (S150) eines geschätzten Gewichts der Testperson unter Verwendung der gemessenen Längen, und
Ausgeben (S170) des berechneten, geschätzten Gewichts,

**dadurch gekennzeichnet, dass**
das Auswählen umfasst:

Extrahieren (S30), aus den dreidimensionalen Daten, eines echoreichen Gebiets, welches ein Gebiet korrespondierend zu den reflektierten Wellen mit einer Reflexionsintensität größer als ein Schwellenwert ist,
Erhalten (S40) von zweidimensionalen Querschnitten, welche die dreidimensionalen Daten bilden, indem die dreidimensionalen Daten basierend auf einem dreidimensionalen Merkmal des extrahierten echoreichen Gebiets geschnitten werden, und

Auswählen (S70) eines der zweidimensionalen Querschnitte als das Messbezugsbild, das zum Messen der Länge des Gebiets in dem Körper der Testperson verwendet wird.

**Revendications**

1.  Appareil de diagnostic par ultrasons (1, 2) comprenant :

    une unité de génération de données tridimensionnelles (105) configurée pour générer des données tridimensionnelles d'une ou plusieurs régions dans le corps d'un sujet sur la base d'ondes réfléchies renvoyées depuis le corps du sujet après que des ondes ultrasonores ont été transmises en direction du corps du sujet ;
    une unité de sélection d'image de mesure (106a) configurée pour sélectionner, sur la base d'une intensité des ondes réfléchies, l'une des sections transversales bidimensionnelles qui composent les données tridimensionnelles, comme image de référence de mesure utilisée pour mesurer une longueur de chaque région dans le corps du sujet ;
    une unité de mesure et de calcul (112) configurée pour mesurer la longueur de chaque région dans le corps du sujet en utilisant l'image de référence de mesure sélectionnée, et pour calculer un poids estimé du sujet en utilisant les longueurs mesurées ; et
    une unité de sortie (113) configurée pour émettre le poids estimé calculé,

    **caractérisé en ce que**
    ladite unité de sélection d'image de mesure (106a) comporte :

    une unité d'extraction de région hyperéchoïque (106) configurée pour extraire, à partir des données tridimensionnelles, une région hyperéchoïque qui est une région correspondant aux ondes réfléchies ayant une intensité de réflexion qui est supérieure à une valeur seuil ;
    une unité d'obtention de plan en coupe (107) configurée pour obtenir des sections transversales bidimensionnelles qui composent les données tridimensionnelles, en coupant les données tridimensionnelles sur la base d'une caractéristique tridimensionnelle de la région hyperéchoïque extraite ; et
    une unité de sélection d'image de référence (108) configurée pour sélectionner l'une des sections transversales bidimensionnelles comme image de référence de mesure utilisée pour mesurer la longueur de la région dans le corps du sujet.

2.  Appareil de diagnostic par ultrasons (1, 2) selon la revendication 1,
    dans lequel ladite unité d'obtention de plan en coupe (107) est configurée pour déterminer, sur la base d'une forme tridimensionnelle et d'un emplacement de la région hyperéchoïque extraite, une orientation d'une section transversale bidimensionnelle dans laquelle les données tridimensionnelles sont coupées, et pour obtenir des sections transversales bidimensionnelles dans l'orientation déterminée.

3.  Appareil de diagnostic par ultrasons (2) selon la revendication 1 ou la revendication 2, comprenant en outre :

    une unité de spécification de région de corps d'un sujet (212) configurée pour spécifier la région, dans le corps du sujet, qui correspond aux données tridimensionnelles,
    dans lequel ladite unité d'obtention de plan en coupe (107) est configurée pour obtenir des sections transversales bidimensionnelles sur la base d'informations indiquant une forme tridimensionnelle et un emplacement de la région spécifiée par ladite unité de spécification de la région de corps du sujet et également sur la base de la forme tridimensionnelle et de l'emplacement de la région hyperéchoïque extraite.

4.  Appareil de diagnostic par ultrasons (2) selon la revendication 3,
    dans lequel ladite unité de spécification de région de corps du sujet (212) est configurée pour spécifier que la région dans le corps du sujet est au moins l'une parmi la tête, l'abdomen et la cuisse, la région correspondant aux données tridimensionnelles.

5.  Appareil de diagnostic par ultrasons (2) selon la revendication 4, comprenant en outre :

    une unité de réception d'opération (110) configurée pour recevoir une instruction d'un opérateur,
    dans lequel ladite unité de spécification de région de corps du sujet (212) est configurée pour spécifier la région dans le corps du sujet selon les instructions de l'opérateur reçues par ladite unité de réception d'opération, la région correspondant aux données tridimensionnelles.

6. Appareil de diagnostic par ultrasons (2) selon la revendication 4,
dans lequel ladite unité de spécification de région de corps du sujet (212) est configurée pour spécifier la région dans le corps du sujet sur la base de la forme tridimensionnelle de la région hyperéchoïque extraite, la région correspondant aux données tridimensionnelles.

7. Appareil de diagnostic par ultrasons (2) selon la revendication 5 ou la revendication 6
dans lequel dans le cas où ladite unité de spécification de région de corps du sujet (212) spécifie que la région, dans le corps du sujet, qui correspond aux données tridimensionnelles, est la tête, ladite unité d'obtention de plan en coupe (107) est configurée pour : extraire une région d'un septum pellucidum sur la base des caractéristiques tridimensionnelles de la région hyperéchoïque ; déterminer, sur la base de la région extraite, une orientation de section transversale bidimensionnelle dans laquelle les données tridimensionnelles sont coupées ; et obtenir des sections transversales bidimensionnelles dans l'orientation déterminée.

8. Appareil de diagnostic par ultrasons (2) selon la revendication 5 ou la revendication 6,
dans lequel dans le cas où ladite unité de spécification de région de corps du sujet (212) spécifie que la région, dans le corps du sujet, qui correspond aux données tridimensionnelles, est l'abdomen, ladite unité d'obtention de plan en coupe (107) est configurée pour : extraire une région d'une colonne vertébrale sur la base des caractéristiques tridimensionnelles de la région hyperéchoïque ; déterminer, sur la base de la région extraite, une orientation de section transversale bidimensionnelle dans laquelle les données tridimensionnelles sont coupées ; et obtenir des sections transversales bidimensionnelles dans l'orientation déterminée.

9. Appareil de diagnostic par ultrasons (2) selon la revendication 5 ou la revendication 6
dans lequel dans le cas où ladite unité de spécification de région de corps du sujet (212) spécifie que la région, dans le corps du sujet, qui correspond aux données tridimensionnelles, est la cuisse, ladite unité d'obtention de plan en coupe (107) est configurée pour : extraire une région d'un fémur sur la base des caractéristiques tridimensionnelles de la région hyperéchoïque ; déterminer, sur la base de la région extraite, une orientation de section transversale bidimensionnelle dans laquelle les données tridimensionnelles sont coupées ; et obtenir des sections transversales bidimensionnelles dans l'orientation déterminée.

10. Appareil de diagnostic par ultrasons (1, 2) selon la revendication 1,
dans lequel ladite unité de sélection d'image de référence (108) est configurée pour sélectionner l'une des sections transversales bidimensionnelles comme image de référence de mesure en évaluant un degré de similitude entre une caractéristique de distribution spatiale d'information de luminosité représentée par chacune des sections transversales bidimensionnelles et une caractéristique de distribution spatiale d'information de luminosité représentée par l'image de référence de mesure.

11. Procédé de traitement d'image comprenant :

la génération (S20, S110) de données tridimensionnelles pour une région dans un corps d'un sujet, sur la base d'ondes réfléchies renvoyées depuis le corps du sujet après que des ondes ultrasonores ont été transmises en direction du corps du sujet ;
la sélection (S70, S130), sur la base d'une intensité des ondes réfléchies, de l'une des sections transversales bidimensionnelles qui composent les données tridimensionnelles, comme image de référence de mesure utilisée pour mesurer une longueur de chaque région dans le corps du sujet ;
la mesure de la longueur de chaque région dans le corps du sujet en utilisant l'image de référence de mesure sélectionnée, et le calcul (S150) d'un poids estimé du sujet en utilisant les longueurs mesurées ; et
l'émission (S170) du poids estimé calculé,

caractérisé en ce que
ladite sélection comporte :

l'extraction (S30), à partir des données tridimensionnelles, d'une région hyperéchoïque qui est une région correspondant aux ondes réfléchies ayant une intensité de réflexion qui est supérieure à une valeur seuil ;
l'obtention (S40) de sections transversales bidimensionnelles qui composent les données tridimensionnelles, en coupant les données tridimensionnelles sur la base d'une caractéristique tridimensionnelle de la région hyperéchoïque extraite ; et

la sélection (S70) de l'une des sections transversales bidimensionnelles comme image de référence de mesure utilisée pour mesurer la longueur de la région dans le corps su sujet.

FIG. 1

## FIG. 2

## FIG. 3

FIG. 4

Pelvis

Thighbone

FIG. 5

Median line

Septum pellucidum

Skull

## FIG. 6

Umbilical vein

Aorta

Spine

Stomach

## FIG. 7A

Thighbone

FL

## FIG. 7B

Incorrect FL

## FIG. 8

START

S10
Generate B-mode images

S20
Generate three-dimensional data

S30
Extract three-dimensional features of hyperechoic region

S40
Obtain cut planes

S50
Evaluate cut planes

S60
Evaluation of cut planes is ended?

S70
Select measurement reference image

S80
Register measurement reference image

END

# FIG. 9

```
        START
          │
          ▼
┌─────────────────────────────────┐
│ Generate three-dimensional data │─── S110
│ which corresponds to region in  │
│ body of subject                 │
└─────────────────────────────────┘
          │
          ▼
┌─────────────────────────────────┐
│ Select measurement reference    │─── S130
│ image based on three-dimensional│
│ data                            │
└─────────────────────────────────┘
```

S100

```
┌─────────────────────────────────┐
│ Calculate estimated weight of   │─── S150
│ subject                         │
└─────────────────────────────────┘
          │
          ▼
┌─────────────────────────────────┐
│ Output estimated weight         │─── S170
└─────────────────────────────────┘
          │
          ▼
         END
```

# FIG. 10

START

S110 — Generate B-mode images — S10

Generate three-dimensional data — S20

Extract three-dimensional features of hyperechoic region in head — S31

Obtain cut planes — S41

Evaluate cut planes — S51

S130 — Evaluation of cut planes is ended? — S61

Select measurement reference image which corresponds to head — S71

Register measurement reference image which corresponds to head — S81

END

# FIG. 11

```
                    ┌─────────────┐
                    │    START    │
                    └──────┬──────┘
                           │
  S110 ┌────────────────────────────────────────────────┐
       │  ┌──────────────────────────────────────┐      │─S10
       │  │      Generate B-mode images          │      │
       │  └──────────────────┬───────────────────┘      │
       │                     │                           │─S20
       │  ┌──────────────────────────────────────┐      │
       │  │   Generate three-dimensional data    │      │
       │  └──────────────────┬───────────────────┘      │
       └────────────────────────────────────────────────┘
       ┌────────────────────────────────────────────────┐─S32
       │  ┌──────────────────────────────────────┐      │
       │  │  Extract three-dimensional features  │      │
       │  │   of hyperechoic region in abdomen   │      │
       │  └──────────────────┬───────────────────┘      │─S42
       │  ┌──────────────────────────────────────┐      │
       │  │          Obtain cut planes           │◄──┐  │
       │  └──────────────────┬───────────────────┘   │  │─S52
       │  ┌──────────────────────────────────────┐   │  │
       │  │         Evaluate cut planes          │   │  │
       │  └──────────────────┬───────────────────┘   │  │
 S130  │             ╱───────┴────────╲              │  │
       │            ╱  Evaluation of    ╲             │  │
       │           ╱   cut planes is     ╲────────────┘  │
       │           ╲     ended?         ╱               │─S62
       │            ╲                  ╱                │
       │             ╲────────┬───────╱                │
       │  ┌──────────────────────────────────────┐      │─S72
       │  │  Select measurement reference image  │      │
       │  │     which corresponds to abdomen     │      │
       │  └──────────────────┬───────────────────┘      │
       │  ┌──────────────────────────────────────┐      │─S82
       │  │ Register measurement reference image │      │
       │  │    which corresponds to abdomen      │      │
       │  └──────────────────┬───────────────────┘      │
       └────────────────────────────────────────────────┘
                           │
                    ┌──────┴──────┐
                    │     END     │
                    └─────────────┘
```

FIG. 12

```
                    ┌──────────────┐
                    │    START     │
                    └──────┬───────┘
                           │
     ┌─────────────────────┼────────────────────────┐
S110 │          ┌──────────▼───────────────┐        │
     │          │  Generate B-mode images  │────────┼── S10
     │          └──────────┬───────────────┘        │
     │          ┌──────────▼───────────────┐        │
     │          │ Generate three-dimensional data │──┼── S20
     │          └──────────┬───────────────┘        │
     └─────────────────────┼────────────────────────┘
     ┌─────────────────────┼────────────────────────┐
     │   ┌──────────────────▼──────────────────┐     │
     │   │ Extract three-dimensional features of │───┼── S33
     │   │   hyperechoic region in thigh        │    │
     │   └──────────────────┬──────────────────┘     │
     │   ┌──────────────────▼──────────────────┐     │
     │   │         Obtain cut planes            │◄───┼── S43
     │   └──────────────────┬──────────────────┘     │
     │   ┌──────────────────▼──────────────────┐     │
     │   │        Evaluate cut planes           │────┼── S53
     │   └──────────────────┬──────────────────┘     │
S130 │              ┌───────▼────────┐               │
     │              │ Evaluation of cut │────────────┤
     │              │ planes is ended?  │            │
     │              └───────┬────────┘               │── S63
     │   ┌──────────────────▼──────────────────┐     │
     │   │ Select measurement reference image   │─────┼── S73
     │   │   which corresponds to thigh         │     │
     │   └──────────────────┬──────────────────┘     │
     │   ┌──────────────────▼──────────────────┐     │
     │   │ Register measurement reference image │─────┼── S83
     │   │   which corresponds to thigh         │     │
     │   └──────────────────┬──────────────────┘     │
     └─────────────────────┼────────────────────────┘
                    ┌──────▼───────┐
                    │     END      │
                    └──────────────┘
```

## FIG. 13

Probe — 101

Transmission and reception unit — 103

B-mode image generation unit — 104

Three-dimensional data generation unit — 105

Hyperechoic region extraction unit — 106

Cut plane obtainment unit — 107

Measurement reference image selection unit — 108

Subject's body region specification unit — 212

106a

Control unit — 102

200

2

Output unit — 113

Measurement and calculation unit — 112

Data storage unit — 109

Operation receiving unit — 110

Display unit — 111

EP 2 623 033 B1

# FIG. 14

START

Generate B-mode images  ⟋S10

Generate three-dimensional data  ⟋S20

Extract three-dimensional features of hyperechoic region  ⟋S30

Specify region in body of subject  ⟋S35

Obtain cut planes  ⟋S40

Evaluate cut planes  ⟋S50

Evaluation of cut planes is ended?  ⟋S60

Select measurement reference image  ⟋S70

Register measurement reference image  ⟋S80

END

## FIG. 15

## FIG. 16

**EP 2 623 033 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H9308630 A
- US 6575907 B1